# EUROPEAN PATENT APPLICATION

(11) **EP 1 938 900 A1**
(43) Date of publication of application: **02.07.2008**
(21) Application number: 07025084.0
(22) Date of filing: 24.12.2007
(51) Int. Cl.: B01L 3/00, B01L 11/00

(54) **Biochip kit and method of analyzing biological sample**

(30) Priority: 29.12.2006 KR 20060137673
(71) Applicant: Samsung Electronics Co., Ltd., Suwon-si, Gyeonggi-Do (KR)
(72) Inventor: Lee, June-Young, Anyang-si Gyeonggi-do (KR); Lee, Dong-Ho, Seongnami-si Gyeonggi-do (KR)
(74) Representative: Weller, Erich W.

(57) **Abstract**

The invention relates to a biochip kit including a biochip disposed in a housing and a method of analyzing a biological sample using the biochip kit.

According to the invention the biochip kit comprises a biochip (130) disposed in a housing (110) and comprising at least one probe and a lid (120) connectedly installed on the housing such that the lid is capable to open or close the housing within a predetermined spatial range.

Use for analyzing biological samples.

## Description

The invention relates to a biochip kit including a biochip disposed in a housing and a method of analyzing a biological sample using the biochip kit.

In recent years, with the advance of genome projects, the genomic nucleotide sequences of various organisms have been identified. Thus, there has been an increasing interest in biochips. Various kinds of biochips have been fabricated in the form of kits, and such biochip kits have been used to analyze various biological samples. Biochip kits that are currently widely available include a hermetically sealed integrated housing and a biochip located in the housing. The housing serves to prevent contamination and damage to the biochip.

According to a representative method for analyzing a biological sample using a biochip kit, a target sample containing target molecules labeled with a fluorescent material is supplied to a biochip so that the target molecules react with probes on the biochip. Then, the biochip is illuminated with light, and emission of light from the fluorescent material is analyzed. Recently, as the design rule of such chips is scaled down, an optical detecting lens is provided closer to a biochip in order to sufficiently collect light emitted from a fluorescent material.

With respect to currently available biochip kits, however, there is a limit to how much the distance from an optical detecting lens to a biochip can be decreased due to the thickness of the housing. Moreover, since light generated in a housing must be detected outside the housing, at least a portion of the housing is formed of a transparent material, e.g., glass. In this regard, however, the transparent material may alter the optical path or wavelength of emitted light. Accordingly, emission of sufficient light cannot be ensured, and it is quite difficult to reliably detect the wavelength of the emitted light.

It is the technical problem underlying the invention to provide a biochip kit and a method for analyzing a biological sample which are capable to reduce or avoid the inconveniencies of the prior art mentioned above and in particular allow a comparatively simple and reliable analysis of the sample.

The invention achieves this object by providing a biochip kit having the features of claim 1 and an analyzing method having the features of claim 12. Advantageous embodiments of the invention are mentioned in the dependent claims the wording of which is herewith incorporated by reference to avoid unnecessary text repetition. The invention thus provides a biochip kit capable of reliably detecting the binding of target molecules in a biological sample to probes on a biochip.

Advantageous embodiments of the invention are described in the following and shown in the drawings in which:
FIG. 1 is a perspective view illustrating a biochip kit;
FIG. 2 is a sectional view taken along a line II - II' of FIG. 1;
FIG. 3 is a perspective view illustrating a biochip kit operating in a folder mode;
FIG. 4A is a perspective view illustrating a biochip kit operating in a sliding mode;
FIG. 4B is a bottom perspective view of a lid of FIG. 4A;
FIG. 5A is a perspective view illustrating a biochip kit operating in a rotational mode;
FIG. 5B is a sectional view illustrating an area A of FIG. 5A;
FIG. 6 is a perspective view illustrating another biochip kit;
FIG. 7 is a sectional view taken along a line VII-VII' of FIG. 6;
FIG. 8 is a perspective view illustrating another biochip kit;
FIG. 9 is a sectional view taken along a line IX-IX' of FIG. 8;
FIGS. 10 through 14 are sectional views illustrating biochips received in corresponding biochip kits;
FIG. 15 is a flowchart illustrating a method of analyzing a biological sample; and
FIG. 16 is a flowchart illustrating another method of analyzing a biological sample.

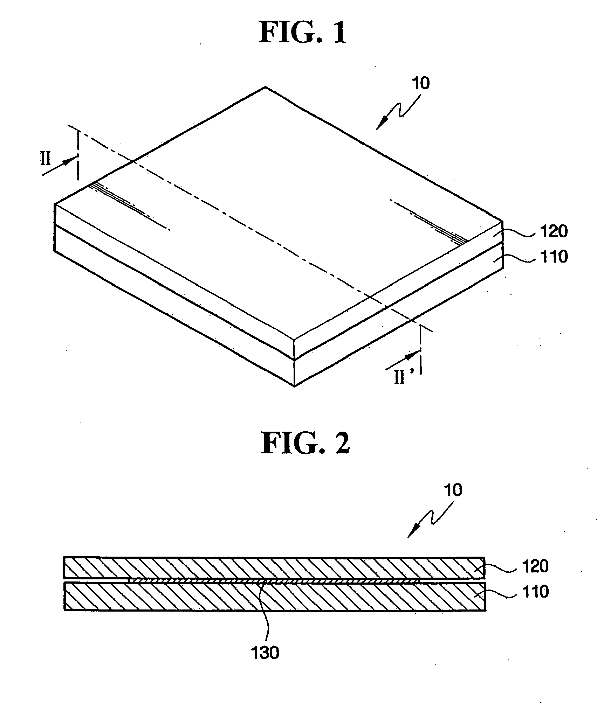

Referring to FIGS. 1 and 2, a biochip kit 10 according to an embodiment of the invention includes a housing 110, a biochip 130 disposed in the housing 110, and a lid 120 connectedly installed on the housing 110. The housing 110 serves as a place for receiving the biochip 130, and at the same time, to protect the biochip 130. For example, the housing 110 may be formed of plastic, glass, or metal such as steel or stainless steel. In order to efficiently protect the biochip 130 from an external impact, the housing 10 may be formed of a rigid material, but is not limited thereto. The biochip 130 may be fixedly placed in the housing 110 regardless of the open/close condition of the lid 120. In order to fix the biochip 130 to the housing 110, an adhesive, etc. may be used.

The biochip 130 can be used in, for example, gene expression profiling, genotyping, detection of mutation or polymorphism such as Single-Nucleotide Polymorphism (SNP), a protein or peptide assay, potential drug screening, development and preparation of novel drugs, etc. For example, the biochip 130 may be a DNA chip or a protein chip. The biochip 130 will be described in more detail later.

The lid 120 is connectedly installed on the housing 110 for opening or closing the housing 110. That is, the lid 120 is not fixed to the housing 110, but can be opened or closed relative to the housing 110 within a predetermined spatial range. As illustrated in FIGS. 1 and 2, when the housing 110 is closed with respect to lid 120, the biochip 130 is shielded. Thus, the lid 120, together with the housing 110, serves to protect the biochip 130. Like the housing 110, the lid 120 may be formed of a polymer, glass, or metal such as steel or stainless steel. Further, the lid 120 may be formed of the same material as the housing 110. In addition, the lid 120 may be formed of a flexible material, e.g., synthetic fiber, non-woven fabric, or soft polymer.

When the housing 110 is closed by the lid 120, a lower surface of the lid 120 can closely contact with an upper surface of the biochip 130. When it is necessary to avoid a contact of the lid 120 with the biochip 130, there may be used a spacer (not shown) for separating the lid 120 from the biochip 130 by a predetermined distance so that the lower surface of the lid 120 does not contact with the upper surface of the biochip 130. The spacer may be extended from the lid 120 and/or the housing 110 or may be attached to the lid 120 and/or the housing 110. The spacer may be a sidewall (not shown) extended from the lid 120 and/or the housing 110. For example, sidewalls may be formed along four edges of the lid 120 and/or the housing 110. In this case, when the sidewalls have the same height and are continuously formed without being disconnected, a space defined between the housing 110 and the lid 120 can be sealed.

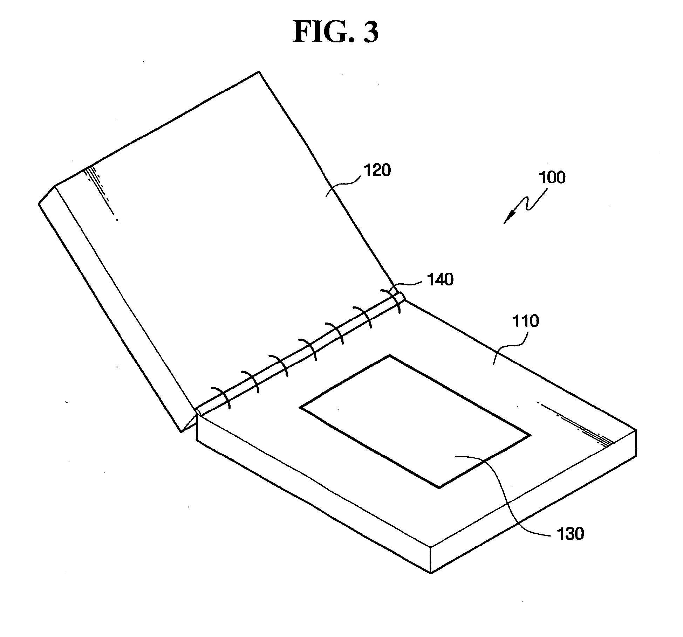

As described above, the lid 120 operates relative to the housing 110 within a predetermined spatial range to open or close the housing 110. Various ways of opening or closing the housing 110 are illustrated in FIGS. 3 through 5B.

Referring to FIG. 3, in a biochip kit 100 according to an embodiment of the invention, the housing 110 is opened or closed in a folding mode. That is, as illustrated in FIG. 3, a side of the lid 120 is joined to the housing 110 and the lid 120 is folded or unfolded along the side of the lid 120 joined to the housing 110. In order to achieve the folding mode operation of the lid 120, the biochip kit 100 may include a joint member 140 for joining a side of the lid 120 to a side of the housing 110. The joint member 140 may be wire, tape, an adhesive, or the like. In addition, the joint member 140 may be any member suitable to achieve the folding mode operation.

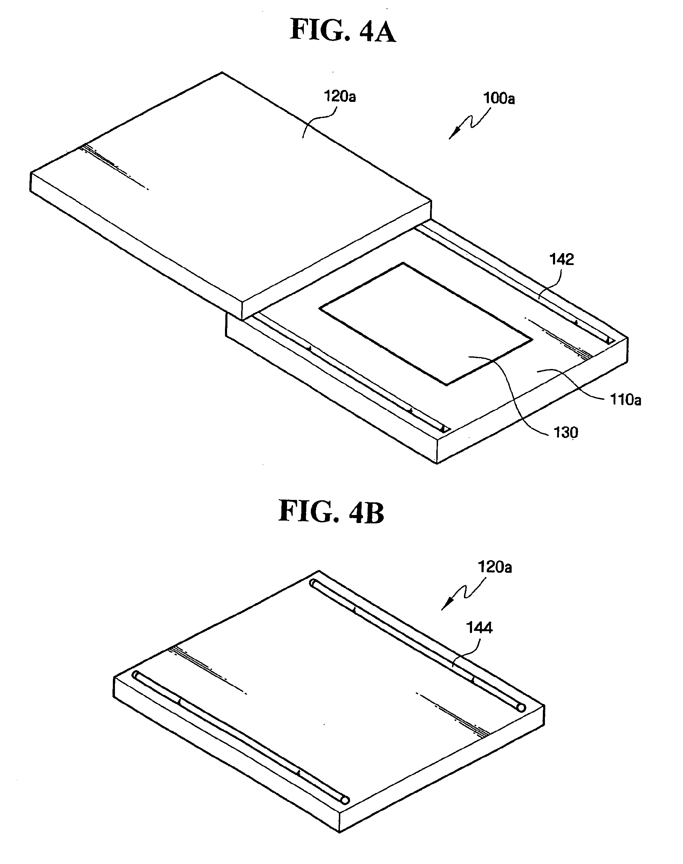

Referring to FIGS. 4A and 4B, in a biochip kit 100a according to an embodiment of the invention, a housing 110a is opened or closed in a sliding mode. That is, as illustrated in FIG. 4A, a lid 120a is disposed to be slidably guided from a side to another. In order to achieve the sliding-mode operation of the lid 120a, the housing 110a may have sliding grooves 142 on the upper sides thereof. The lid 120a may include line-type sliding protrusions 144 corresponding to the sliding grooves 142 so that the sliding protrusions 144 are slidably received in the sliding grooves 142. Although not shown, according to an alternative version of the current embodiment of the present invention, a housing may include line-type sliding protrusions and a lid may have sliding grooves corresponding to the sliding protrusions.

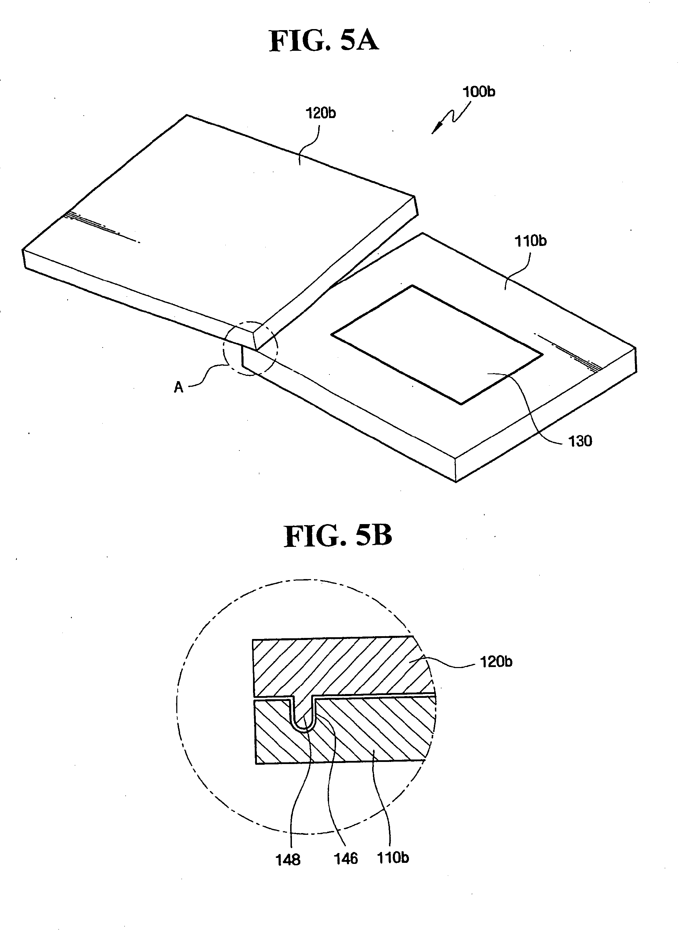

In a biochip kit 100b according to another embodiment of the invention, a housing 110b is opened or closed in a rotating mode. That is, as illustrated in FIG. 5A, a lid 120b can be pivotably operated in a state wherein an end of the lid 120b is fixed to the housing 110b. In order to achieve the rotating operation of the lid 120b, as illustrated in FIG. 5B, the housing 110b may have a groove 146 on an upper end thereof, and the lid 120b may include a pivotable protrusion or shaft 148 corresponding to the groove 146 so that the pivotable protrusion or shaft 148 can be rotated in the groove 146. Although not shown, according to an alternative version of the current embodiment of the present invention, a housing may include a pivotable protrusion or shaft and a lid may have a groove corresponding to the protrusion or shaft.

As described above with reference to FIGS. 1 through 5, when a housing 110, 110a, or 110b is opened by moving a lid 120, 120a, 120b in a folding mode, a sliding mode, or a rotating mode, an upper surface of a biochip 130 disposed in the housing is directly exposed to outside. As such, when an upper surface of the biochip is directly exposed to outside, a detector (not shown) can be disposed to be as close as possible to the upper surface of the biochip.

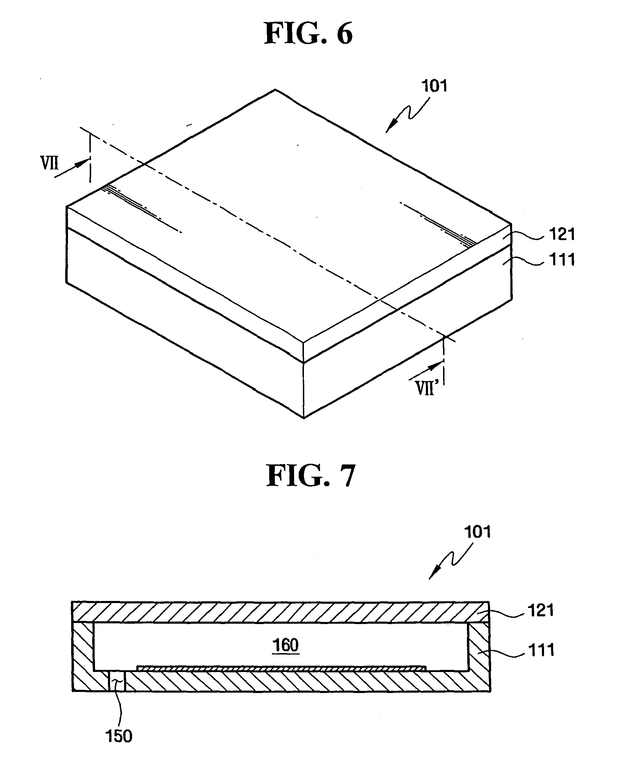

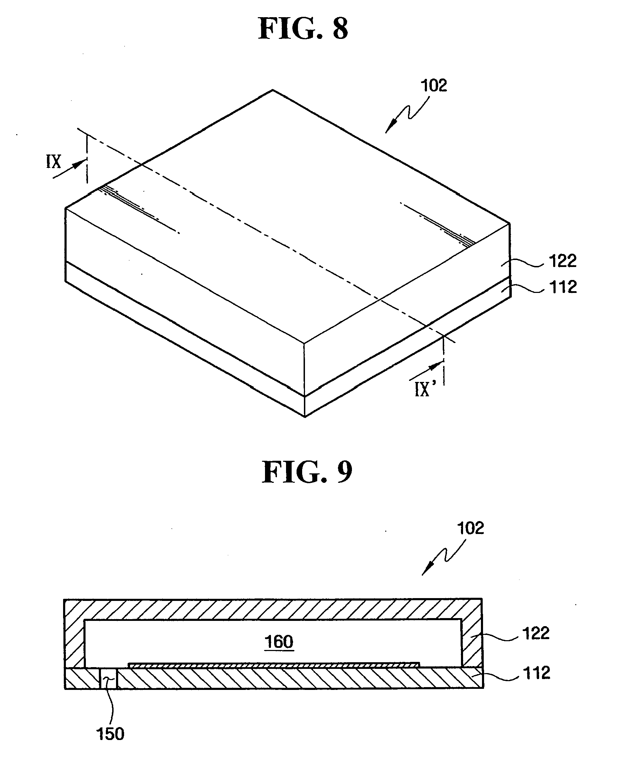

Referring to FIGS. 6 through 9, in biochip kits 101 and 102 according to further embodiments of the invention, unlike the previous embodiments described above, when housings 111 and 112 are closed with lids 121 and 122, lower surfaces of the lids 121 and 122 are not contacted to an upper surface of the biochip 130 but are separated from the upper surface of the biochip 130 by a predetermined distance. Thus, a reaction space 160 is defined between the lids 121 and 122 and the biochip 130. For this, sidewalls may be extended from the housing 111, as illustrated in FIGS. 6 and 7, or may be extended from the lid 122, as illustrated in FIGS. 8 and 9. The height of the sidewalls affects the capacity of the reaction space 160, and thus, can be appropriately determined by the desired reaction conditions, the amount of a sample which is introduced in the reaction space 160, etc.

The reaction space 160 provides a place where a binding reaction between a target molecule in a biological sample and a probe on the biochip 130, e.g., a target-probe hybridization reaction can occur. In order to prevent an external contamination and to easily control reaction conditions during a target-probe binding reaction, the reaction space 160 may be hermetically sealed. That is, when the housings 111 and 112 are closed by the lids 121 and 122, internal spaces defined by the lids 121 and 122 and the housings 111 and 112 can be hermetically sealed.

Meanwhile, in order to promote the above-described target-probe binding reaction, the housings 111 and 112 may further include at least one port 150 for permitting entry or exhaust of a fluid, e.g., a biological sample, a cleaning solution, or a nitrogen gas. For example, a single port can be responsible for both entry and exhaust of a fluid, or alternatively, at least one of two or more ports can serve as an inlet port for entry of a fluid and at least another port can serve as an outlet port for exhaust of a fluid. Furthermore, according to an alternative version of the present embodiment, an inlet/outlet port as described above may be provided in a lid or both in a housing and in a lid.

The folding mode, sliding-mode, or rotating mode lid described above with reference to FIGS. 3 through 5B can also be applied to the biochip kits described with reference to FIGS. 6 through 9. Thus, with respect to the biochip kits described with reference to FIGS. 6 through 9, it should be understood that a detector can be disposed to be as close as possible to an upper surface of a biochip.

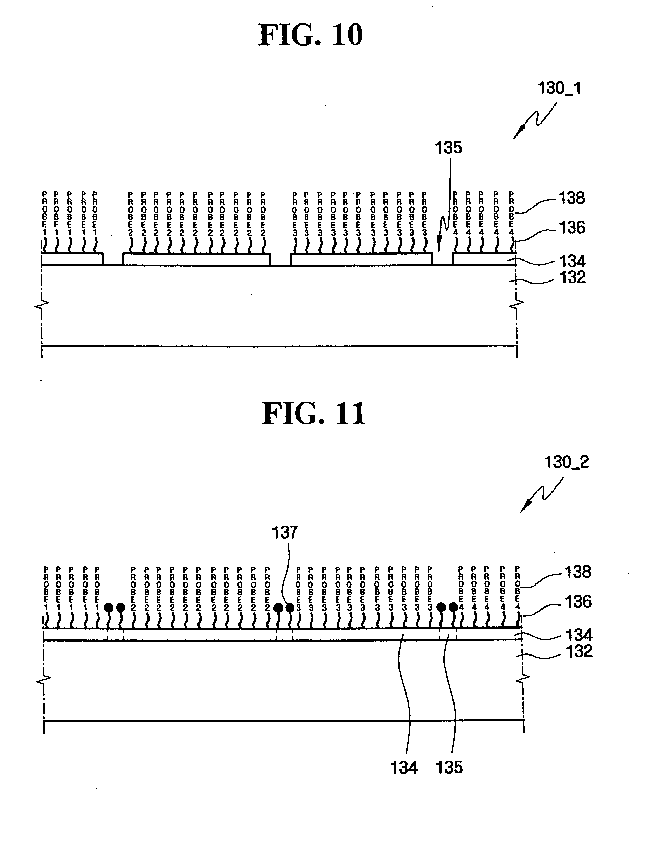

Hereinafter, biochips that can be applied to biochip kits according to embodiments of the invention will be described in detail. FIGS. 10 through 14 are sectional views illustrating biochips received in biochip kits according to embodiments of the invention. Referring to FIGS. 10 through 14, biochips 130_1, 130_2, 130_3, 130_4, and 130_5 include a substrate 132, and active regions 134 disposed on the substrate 132, a plurality of probes 138 being immobilized on the active regions 134.

The substrate 132 may be a flexible or a rigid substrate. Examples of a flexible substrate to be used include a nylon membrane, a nitrocellulose membrane, a polymeric film, etc. A rigid substrate may be a semiconductor substrate, a transparent glass substrate formed of soda lime glass, etc. In biochip kits according to embodiments of the invention, the analysis of the biochips 130_1, 130_2, 130_3, 130_4, and 130_5 can be directly performed on upper surfaces of the biochips 130_1, 130_2, 130_3, 130_4, and 130_5. Thus, even when an opaque substrate such as a semiconductor substrate, a membrane, or a polymeric film is used as the substrate 132, the biochips 130_1, 130_2, 130_3, 130_4, and 130_5 can be easily analyzed. In particular, when a semiconductor substrate is used as the substrate 132, it is advantageous to use a semiconductor device fabrication process, various thin film formation processes which have been stably established and applied, a photolithography process, etc.

The active regions 134 are disposed on the substrate 132. The active regions 134 may be formed of a material that is substantially stable against hydrolysis upon hybridization assay, e.g., upon contacting with a pH 6-9 phosphate or Tris buffer. For example, the active regions 134 may be formed of silicone oxide such as plasma-enhanced tetraethylorthosilicate (PE-TEOS), high-density plasma (HDP) oxide, P-SiH4 oxide, or thermal oxide; silicate such as hafnium silicate or zirconium silicate; metal oxinitride such as silicon oxinitride, hafnium oxinitride, or zirconium oxinitride; metal oxide such as titanium oxide, tantalum oxide, aluminum oxide, hafnium oxide, zirconium oxide, or ITO; polyimide; polyamine; metal such as gold, silver, copper, or palladium; or a polymer such as a polystyrene, a polyacrylic acid, or a polyvinyl compound.

The active regions 134 are isolated from each other by probe cell isolation regions 135. The active regions 134 and the probe cell isolation regions 135 are arranged according to presence or absence of the probes 138. A plurality of the probes 138 are coupled onto the active regions 134. For example, the same probes are coupled onto each active region and probes coupled onto an active region may be different from probes coupled onto another active region. The probes 138 can be diversely selected according to a biological sample sought to be analyzed. For example, the probes 138 may be oligomer probes.

In one embodiment, the term "oligomer" can be a low-molecular weight polymer molecule comprising two or more covalently bound monomers. In another embodiment, oligomers can have a molecular weight of up to about 1,000, but the embodiments of the invention are not limited thereto. The oligomer may include from about 2 up to 500 monomers, preferably from about 5 up to 30 monomers. The monomers may be, for example, nucleosides, nucleotides, amino acids, peptides, etc. according to the type of probes. In a further embodiment, previously synthesized oligomer probes may be coupled to active regions, or oligomer probes may be synthesized on active regions by in-situ photolithography.

In another emodiment, the terms "nucleosides" and "nucleotides" can include purine and pyrimidine bases, and in still another embodiment, methylated purines or pyrimidines, acylated purines or pyrimidines, etc. Furthermore, the "nucleosides" and "nucleotides" can include (deoxy)ribose, but also, as well as modified sugars which contain a halogen atom or an aliphatic group substituted for at least one hydroxyl group or functionalized with an ether, an amine, or the like.

In a further emodiment, the term "amino acids" can include not only naturally occurring, L-, D-, and nonchiral amino acids, but also to, for example, modified amino acids, amino acid analogs, etc. In other embodiments, the term "peptides can include compounds produced by, for example, an amide bond between the carboxyl group of one amino acid and an amino group of another amino acid. Accordingly, the probes 138 may comprise two or more nucleosides, nucleotides, amino acids, peptides, or the like.

The coupling between the active regions 134 and the probes 138 can be achieved by means of linkers 136 which are interposed between the active regions 134 and the probes 138.

Referring to FIG. 10 illustrating the biochip 130_1 according to some embodiments of the invention, the active regions 134 are patterned. Here, active region-free exposed portions of the substrate 132 are defined as the probe cell isolation regions 135.

Referring to FIG. 11 illustrating the biochip 130_2 according to another embodiment of the invention, the active regions 134 are not physically patterned but are defined by the probe cell isolation regions 135 which are not coupled with the probes 138 but coupled with inactivated linkers 137.

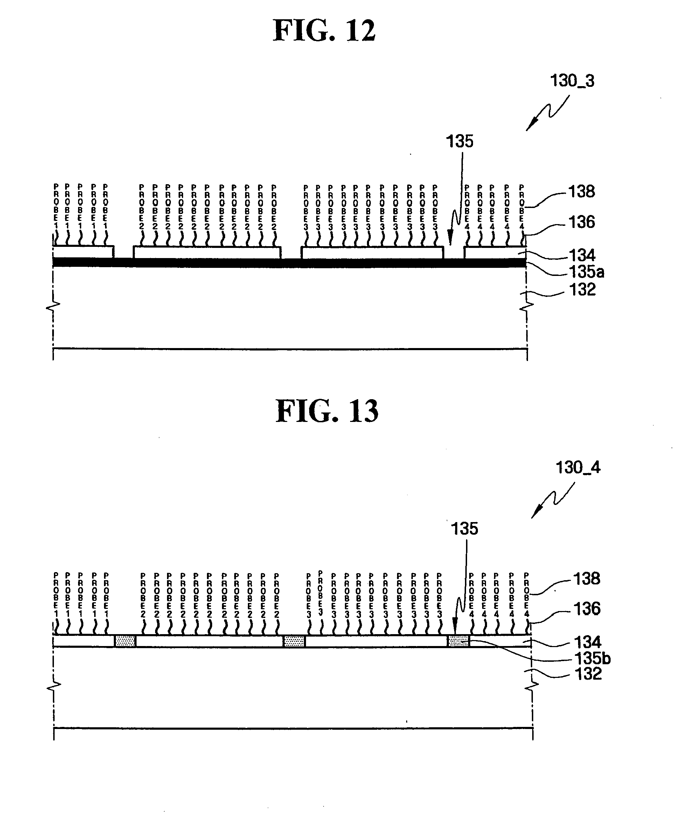

Referring to FIG. 12 illustrating the biochip 130_3 according to still another embodiment of the invention, a coupling blocking film 135a is disposed on the substrate 132, and the active regions 134 are physically patterned on the coupling blocking film 135a. The coupling blocking film 135a may be formed of fluorine-containing fluoride such as fluorosilane. Here, active region-free upper portions of the coupling blocking film 135a are defined as the probe cell isolation regions 135.

Referring to FIG. 13 illustrating the biochip 130_4 according to a further embodiment of the invention, the active regions 134 are patterned as illustrated in FIG. 10, and spaces defined between the active regions 134 are filled with blocking fillers 135b. The blocking fillers 135b may be formed of a material having characteristics preventing the coupling of the probes 138 and good gap-filling characteristics, e.g., fluorosilane or polysilicone. Here, upper portions of the blocking fillers 135b are defined as the probe cell isolation regions 135.

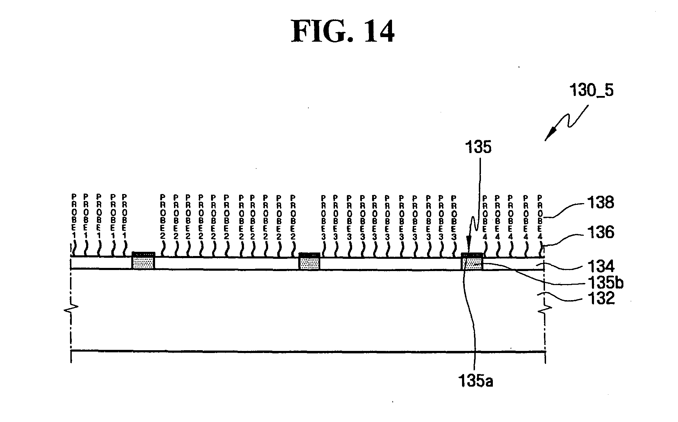

Referring to FIG. 14 illustrating the biochip 130_5 according to still a further embodiment of the invention, the active regions 134 have substantially the same structure as illustrated in FIG. 13, but coupling blocking film patterns 135a are disposed on blocking fillers 135b filled in spaces defined between the active regions 134. Thus, upper portions of the coupling blocking film patterns 135a are defined as the probe cell isolation regions 135.

The biochips 130_1, 130_2, 130_3, 130_4, and 130_5 illustrated in FIGS. 10 through 14 may be substituted for biochips (see 130) of biochip kits (see 100, 100a, 100b, 101, and 102) as illustrated in FIGS. 1 through 9.

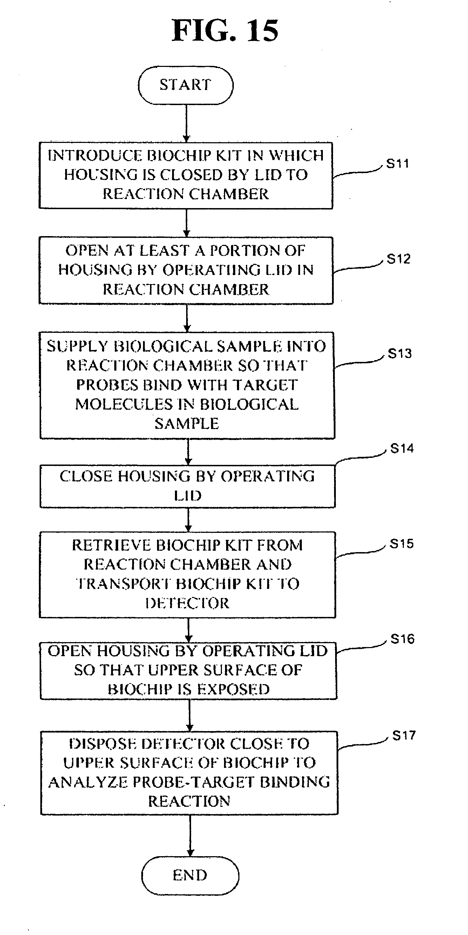

Hereinafter, methods of analyzing biological samples using biochip kits as described above according to embodiments of the invention will be described. FIG. 15 illustrates a method of analyzing a biological sample according to an embodiment of the invention which is related to analysis of a biological sample using a biochip kit with no reaction space as illustrated in FIGS. 1 through 5B. Referring to FIG. 15, together with FIGS. 1 through 5B, a biochip kit (see 10, 100, 100a, 100b) in which a housing (see 110, 110a, 110b) is closed by a lid (see 120, 120a, 120b) is introduced into a reaction chamber (S11). The reaction chamber provides a reaction space where target-probe binding events occur. Thus, the reaction chamber may include an inlet/outlet port for permitting entry or exhaust of a fluid such as a biological sample. Meanwhile, the housing is closed by the lid until the biochip kit is introduced into the reaction chamber. Thus, a biochip (see 130) is shielded with the lid, and thus, can be protected from external contaminants and impact, etc.

Next, the lid is opened in the reaction chamber so that at least a portion of the housing is exposed (S12). That is, at least a portion of the housing is opened by moving the lid in a folding mode, a sliding mode, or a rotating mode, thereby defining a spatial passage reaching an upper surface of the biochip. If the anticontamination function of the reaction chamber is reliable, the lid can be opened until an upper surface of the biochip is directly exposed to outside. Next, a biological sample is supplied into the reaction chamber so that target molecules in the biological sample bind with probes on the biochip (S13). The biological sample which has been supplied into the reaction chamber is directed toward an upper surface of the biochip through the spatial passage defined in the above-described manner. The biological sample is a sample containing target molecules sought to be analyzed, and may contain DNAs. An end of a single-stranded DNA may be labeled with a fluorescent material.

Next, reaction conditions are adjusted so that the probes on the biochip bind with the target molecules of the biological sample. When the probes are oligonucleotide probes and the biological sample contains single-stranded DNAs, a target-probe binding reaction may be a hybridization reaction. Meanwhile, when the biological sample contains unlabeled single-stranded DNAs, supply of a fluorescent material-containing solution into the reaction chamber may be further performed to attach a fluorescent material to probes which have been bound (e.g., hybridized) to the target DNAs.

After the target-probe binding reaction is terminated, the biological sample is discharged from the reaction chamber. Next, optionally, in order to completely remove the biological sample remained on the biochip kit, a cleaning solution may be introduced in the reaction chamber. Although not necessary, in order to dry the upper surface of the biochip, a dry gas may be supplied into the reaction chamber. The dry gas may be a nitrogen gas, etc.

Next, the lid is operated to close the housing (S14). When the housing is closed, the biochip where target-probe binding events have occurred is closed and protected by the lid. Next, the biochip kit is retrieved from the reaction chamber and transported to a detector (not shown) (S15). As described above, since the biochip is closed and protected by the lid, external contamination etc. does not occur during transporting to the detector.

Next, the lid is operated to open the housing, thereby exposing an upper surface of the biochip (S16). For this, the lid can be operated in a folding mode, a sliding mode, or a rotating mode, as described above. In step S16, the lid is operated so that the upper surface of the biochip is completely exposed.

Meanwhile, when the transport of the biochip kit from the reaction chamber to the detector is performed in a closed chamber capable of protecting the biochip kit from contaminants or impact sources, step S 14 and step S16 may be omitted.

Next, the detector is disposed to be close to the biochip to analyze probe-target binding events (S17). The detector may include an optical detector. The optical detector can readily detect target-probe binding events when a biological sample contains a fluorescent material or a fluorescent material-containing solution is used. For example, if target-probe binding events (e.g., hybridization) have occurred and thus a fluorescent material remains on a biochip, when an upper surface of the biochip is illuminated with a first wavelength light, the fluorescent material is excited by the first wavelength light, thereby emitting a second wavelength light different from the first wavelength light. On the other hand, if no target-probe binding events have occurred, and thus no fluorescent material is left on a biochip, light with a different wavelength from the first wavelength is not emitted. In this regard, target-probe binding events can be analyzed by detecting the second wavelength light using the optical detector of the detector.

For example, the optical detector may be a charge coupled device (CCD), a CMOS image sensor (CIS), etc. The lens of the CCD or CIS focuses emitted light and converts the focused light to an electrical signal to analyze the quantity and wavelength of the emitted light. Meanwhile, a second wavelength light emitted from a fluorescent material generally follows a random optical path. Thus, in order to collect the requisite amount of light, it is preferred to place a detector, specifically, the lens of a CCD or CIS in the detector or an objective lens connected thereto, which is located as close as possible to a light-emitting source. In particular, as design rule is scaled down, placing an optical detector in close proximity of a light-emitting source is becoming more important. If a medium is interposed between a light-emitting source and a lens, an optical path is altered according to Snell's Law. In particular, when an interface between the medium and the light-emitting source, or if the lens is uneven, irregularity of the optical path worsens. Furthermore, when light passes from a first medium into a second medium with a different refractive index than the first medium, the wavelength of the light is altered, thereby rendering accurate wavelength detection difficult.

However, in biochip kits according to embodiments of the invention, a lid is operated to open a housing, thereby completely exposing an upper surface of a biochip to outside. Thus, the lens of a CCD or CIS or an objective lens connected thereto can be disposed to be closer to the biochip due to the absence of a medium except air. Therefore, it is possible to sufficiently collect emitted light and accurately detect the wavelength of the emitted light, thereby ensuring more reliable analysis. Even though the upper surface of the biochip is exposed, chip contamination is negligible since target-probe binding events have already been completed.

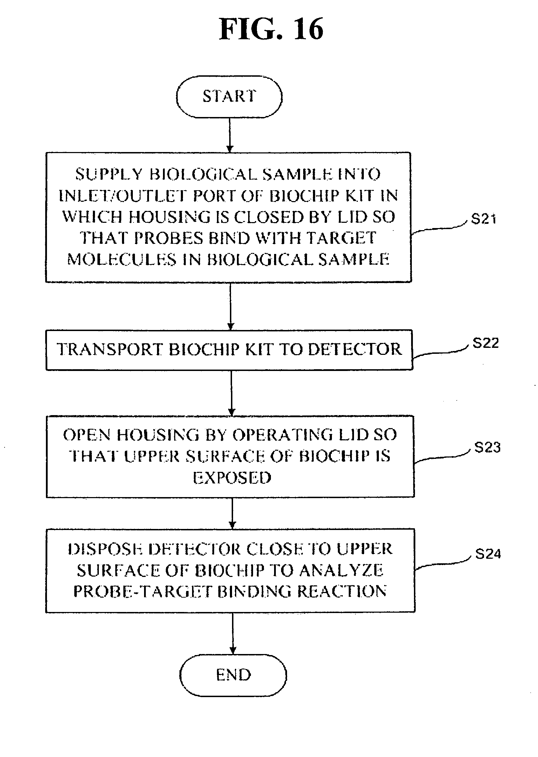

FIG. 16 is a flowchart illustrating a method of analyzing a biological sample according to another embodiment of the invention. This embodiment is related to analysis of a biological sample using a biochip kit including a reaction space as illustrated in FIGS. 6 through 9. An overlapping explanation with the embodiment illustrated in FIG. 15 will be omitted or simplified. That is, the current embodiment of the invention will be described mainly in terms of the difference from the embodiment illustrated in FIG. 15.

Referring to FIG. 16, together with FIGS. 6 through 9, a biological sample is supplied into a biochip kit (see 101, 102) in which a housing (see 111, 112) is closed by a lid (see 121, 122) via an inlet/outlet port (see 150) so that target molecules in the biological sample bind with probes on the biochip kit (S21). That is, the analysis of the biological sample according to the current embodiment of the present invention is performed in a reaction space (see 160) defined in the biochip kit without using a reaction chamber described above with reference to FIG. 15. Since the supply of the biological sample is performed through the inlet/outlet port, it is not necessary to open the housing by operating the lid in step S21.

Next, the biochip kit is transported to a detector (S22). In step S22, the housing is closed by the lid, and thus, an upper surface of a biochip (see 130) received in a space defined by the housing and the lid is not exposed to outside, which is substantially the same as step S15 illustrated in FIG. 15.

Next, the lid is operated to open the housing, thereby exposing the upper surface of the biochip (S23). Step S23 is substantially the same as step S16 illustrated in FIG. 15.

Next, the detector is disposed to be close to the upper surface of the biochip to analyze target-probe binding events (S24). Step S24 is substantially the same as step S 17 illustrated in FIG. 15.

In the analysis of the biological sample according to this embodiment of the invention, target-probe binding events occur in the reaction space defined in the biochip kit in the absence of a reaction chamber, and thus, the analysis of the biological sample can be performed in a more simple and reliable manner.

As apparent from the above description, in biochip kits according to embodiments of the invention, a lid is installed to open or close a housing. Thus, it is possible to cover or expose a biochip disposed in the housing when needed. As such, since a biochip can be closed by a lid, the biochip can be protected from external contaminants and impact. Moreover, when a biochip is exposed to outside by opening a housing by operating a lid, a detector can be disposed to be as close as possible to the biochip. Thus, it is possible to sufficiently collect emitted light and to accurately detect the wavelength of the emitted light, thereby ensuring more reliable analysis.

## Claims

1. A biochip kit for analyzing a biological sample comprising
a biochip (130), disposed in a housing(110) and comprising at least one probe (138); and
a lid (120), connectedly installed on the housing such that the lid is capable to open or close the housing within a predetermined spatial range.

2. The biochip kit of claim 1, wherein the lid closes the housing for the biochip to be covered, and opens the housing for an upper surface of the biochip to be exposed.

3. The biochip kit of claim 2, wherein the lid is connectedly installed on the housing such that the lid is capable to open or close the housing by moving the lid in a predetermined spatial range in a sliding mode, a folding mode, or a rotating mode.

4. The biochip kit of any of claims 1 to 3, wherein the lid closes the housing to define a reaction space (160) together with the housing.

5. The biochip kit of claim 4, wherein at least one of the housing and the lid comprises an inlet/outlet port (150) entering the reaction space.

6. The biochip kit of any of claims 1 to 5, wherein the biochip is fixed to the housing.

7. The biochip kit of any of claims 1 to 6, wherein the biochip comprises:
a substrate (132); and
active regions (134) disposed on the substrate, each probe being immobilized on the active regions.

8. The biochip kit of claim 7, wherein the substrate is a semiconductor substrate, a glass substrate, or a polymer substrate.

9. The biochip kit of claim 7 or 8, wherein each probe is immobilized on the active regions via linkers (136).

10. The biochip kit of any of claims 1 to 9, wherein the active regions are isolated from each other by probe cell isolation regions (135) on which none of the probes are immobilized.

11. The biochip kit of any of claims 1 to 10, wherein the one or more probes are oligonucleotide probes.

12. A method of analyzing a biological sample, the method comprising:
providing a biochip kit according to any of claims 1 to 11,
allowing the at least one probe of the biochip kit to bind with target molecules in the biological sample,
opening the housing by moving the lid so that an upper surface of the biochip is exposed, and
disposing a detector close to the biochip to analyze a probe-target binding reaction.

13. The method of claim 12, wherein the binding of the at least one probe with the target molecules in the biological sample comprises:
opening at least a portion of the housing by moving the lid so that at least a portion of the upper surface of the biochip is exposed; and
supplying the biological sample onto the exposed upper surface of the biochip.

14. The method of claim 12 or 13, wherein the binding of the at least one probe with the target molecules in the biological sample is performed in a reaction chamber comprising an inlet/outlet port.

15. The method of claim 14, wherein the binding of the at least one probe with the target molecules in the biological sample comprises:
supplying the biological sample into the reaction space through the inlet/outlet port; and
allowing each probe to bind with the target molecules in the biological sample in the reaction space.

16. The method of any of claims 12 to 15, wherein the biological sample comprises a fluorescent material, and the analysis of the probe-target binding reaction comprises detecting light emitted from the fluorescent material.

17. The method of any of claims 12 to 16, wherein a charge coupled device (CCD) or a CMOS image sensor (CIS) is used for the detector.

18. The method of any of claims 12 to 17, wherein each probe is a oligonucleotide probe, and the target-probe binding reaction is a hybridization reaction.
